## Europäisches Patentamt
## European Patent Office
## Office européen des brevets

(19)

(11) Publication number: **0 070 266**
**B1**

(12) **EUROPEAN PATENT SPECIFICATION**

(45) Date of publication of patent specification: **15.01.86**

(51) Int. Cl.⁴: **B 65 D 45/00, A 61 M 16/00**

(21) Application number: **82850138.7**

(22) Date of filing: **21.06.82**

(54) **A sealing device.**

(30) Priority: **09.07.81 SE 8104269**

(43) Date of publication of application:
**19.01.83 Bulletin 83/03**

(45) Publication of the grant of the patent:
**15.01.86 Bulletin 86/03**

(84) Designated Contracting States:
**AT BE CH DE FR GB IT LI LU NL**

(56) References cited:
**GB-A- 4 615**
**US-A-1 625 056**
**US-A-2 462 445**

(73) Proprietor: **Mediplast AB**
**Rasundavägen 60**
**S-171 52 Solna (SE)**

(72) Inventor: **Jacob, Peter**
**Violvägen 6**
**S-810 60 Söderfors (SE)**

(74) Representative: **Roth, Ernst Adolf Michael et al**
**GÖTEBORGS PATENTBYRA AB Box 5005**
**S-402 21 Göteborg (SE)**

Courier Press, Leamington Spa, England.

EP 0 070 266 B1

## Description

The present invention relates to a sealing device substantially intended for changing the inner volume of a container, such as containers or packages for chemicals, drugs, etc., and pressure chambers for breathing apparatuses, comprising a base plate, an upper plate and an expansion means or elastic seal provided between the base plate and the upper plate, and a support which is firmly connected to the central part of the upper plate and projects through a recess in the base plate.

A problem in connection with anaesthetic ventilators is to be able to easily adjust the tidal volume, i.e. the volume of the breathing gas which shall be supplied to a patient. According to present technique this is done by for example adjusting the length of stroke of an air pump in such a way that the pump at each stroke provides the volume of gas intended. According to another known procedure a breathing bag of an appropriate size is used for each patient. To adjust the correct length of the stroke of a pump requires a mechanical construction which impairs its reliability in operation. Moreover, it is difficult for the operation staff to regularly and visually control that the correct volume is supplied to the patient.

A closely related problem area is the sealing of containers. There is a great need for adapting the enclosed volume of a container to the volume of the material which is stored in the container. For many purposes, for example in case of containers or packages for storing chemicals and drugs it is desirable and sometimes necessary to reduce to the greatest possible extent the exposure of the chemicals and the drugs to air. When the contents of such a package gradually is reduced this volume is normally replaced by air. For closing or sealing the container screw caps, snap-in lips, clamp caps, ground-in lips, etc. are usually used. To solve this sealing problem it has been proposed to transfer the material remaining after the contents of a package has been reduced to a smaller container, the volume of which being as closely as possible in agreement with the volume of the remaining material. By this it is prevented that the air provides an injurious effect on the material in the container during a long time.

The preamble of Claim 1 is based on US—A—2,462,445.

In US—A—2,462,445 is disclosed an air-tight closure for containers in which solid, liquid or gaseous substances can be stored. The closure comprise two circular plates with a rubber sealing ring between their outer edges. Means in the form of a screwed post and a nut are provided for pressing the plates together whereby the sealing ring will be distorted and squeezed outwardly to seal tightly against the inner periphery of the container. In another embodiment, the sealing is effected by means of a bifurcated lever which is pivotable aobut a horizontal pivot pin. This disclosure has a number of drawbacks. For instance in its first embodiment, one has to rotate a nut which is time consuming and it is hard to know how hard you should rotate the nut. In the embodiment comprising the lever it is difficult to keep the closure at the proper position and manipulate the lever at the same time.

The purpose of the present invention is to eliminate the drawbacks mentioned above and to provide a sealing device which can be easily adjusted and locked in the desired position. With the sealing device one shall further obtain an excellent sealing against surrounding walls. A further purpose is to provide a sealing device which can be manufactured in a simple way and to low costs.

The essential features of the invention are set forth in claim 1.

The invention will be further described below by way of a working example, reference being made to the accompanying drawings, in which

Fig. 1 is a perspective view of a sealing device according to the invention in the form of an adjustable bottom provided in a cut-up cylinder, and

Fig. 2 is a vertical section view of a sealing device according to the invention, the construction of which being shown in greater detail.

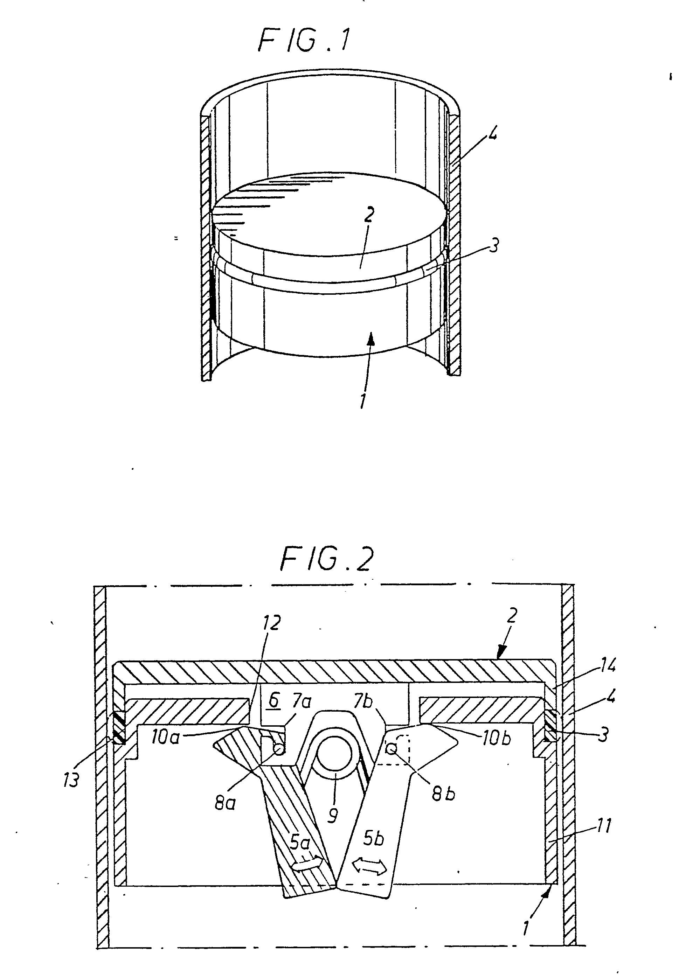

In Fig. 1 there is shown a sealing device according to the invention comprising a cylindrical base plate 1, a cylindrical upper plate 2 and an elastic seal 3 provided between said plates. The sealing device is slidably provided in a cylindrical shell 4 of a container made of a rigid material, such as plastic, metal or the like.

Fig. 2 is a detailed view of the sealing device according to the invention. Two pressure means 5a,b which are formed as levers and which can be manually actuated are pivotly provided at a support 6 fixedly connected with the central part of the upper plate 2. Lever shafts 8a,b which are provided through the pressure means 5a,b are received in recesses 7a,b. Between the pressure means 5a,b there is further provided a resilient means for example a coil spring 9 which is intended to move the longer legs of the pressure means 5a,b from each other. By this a force is exerted on the underside of the base plate 1 at the stop points 10a,b of the pressure means 5a,b which forces the base plate 1 and the upper plate 2 to approach each other. The base plate 1 is provided with a relatively wide circumferential edge 11 which guides the sealing device in the shell.

Further, in the central part of the base plate 1 there is provided a recess 12 intended to permit the displacement of the support 6 fixedly provided in the upper plate 2 through the central part of the base plate 1. At the same level as the lever shafts 8a,b provided on the support 6 there is formed a groove 13 in the edge 11 of the base plate 1 which receives a circular elastic seal 3 for example made of silicon rubber.

The upper plate 2 is also provided with a circumferential edge 14 which can easily run in the groove 13.

The base plate 1, the upper plate 2 and the elastic seal 3 are formed with an outer diameter which is somewhat less than the inner diameter of the shell 4 and due thereto they can be displaced easily in an axial direction when the pressure means are manually inactivated. In activated position, that is when the coil spring 9 via the pressure means 5a,b forces the base plate 1 and the upper plate 2 to approach each other because of the lever effect obtained by the pressure means the elastic seal 3 is expanded in a radial direction. The outer diameter of the seal 3 increases until the seal presses tightly against the inner wall of the shell 4 and locks in this way the sealing device. When the sealing device is inactivated again the base plate 1 retreats from the upper plate 2 because of the fact that the elastic seal 3 endeavours to restore its original form and thus exerts a pressure axially.

The invention is of course not restricted to the example described above but several alternative embodiments are possible within the scope of the invention. Thus, the shell 4 and the sealing device can be formed with another cross section than a cylindrical cross section, for example a square cross section or the like.

**Claims**

1. A sealing device substantially intended for changing the inner volume of a container (4), such as containers or packages for chemicals, drugs, etc., and pressure chambers for breathing apparatuses, comprising a base plate (1), an upper plate (2) and an expansion means or elastic seal (3) provided between the base plate (1) and the upper plate (2), and a support (6) which is firmly connected to the central part of the upper plate (2) and projects through a recess (12) in the base plate (1), characterized in, that two pressure means (5a,b), which are formed as separate levers, are connected to the support (6) and being manually pivotable in opposition to the effect of resilient means (9) which are arranged between the levers to press the plates (1, 2) toward one another thereby to deform the elastic seal (3) into engagement with the wall of the container (4).

2. A device according to claim 1, characterized in, that the support (6) has recesses (7a,b) for receiving lever shafts (8a,b) running through the pressure means (5a,b).

**Patentansprüche**

1. Dichtungsvorrichtung, insbesondere zum Aendern des Innenvolumens eines Behälters (4), beispielsweise Behälter oder Behältnisse für Chemikalien, Arzneimittel, etc. und Druckkammern für Atmungsgeräte, enthaltend eine Grundplatte (1), eine Oberplatte (2) und ein Expansionsmittel oder eine elastische Dichtung (3), welche zwischen der Grundplatte (1) und der Oberplatte (2) angeordnet ist, und einen Träger (6), der mit dem mittleren Bereich der Oberplatte (2) fest verbunden ist und durch eine Ausnehmung (12) in der Grundplatte (1) ragt, dadurch gekennzeichnet, dass zwei Pressmittel (5a,b), die als getrennte Hebel ausgebildet sind, mit dem Träger (6) verbunden und gegen die Wirkung von nachgiebigen Mitteln (9), die zwischen den Hebeln angeordnet sind, manuell schwenkbar sind, um die Platten (1, 2) gegeneinander zu drücken, um damit die elastische Dichtung (3) zur Anlage an die Wand des Behälters (4) zu pressen.

2. Vorrichtung nach Anspruch 1, dadurch gekennzeichnet, dass der Träger (6) Ausnehmungen (7a,b) zur Aufnahme von die Pressmittel (5a,b) durchsetzenden Hebelwellen (8a,b) aufweist.

**Revendications**

1. Dispositif d'étanchéité essentiellement destiné à modifier le volume interne d'un récipient (4), tel que des récipients ou emballages pour produits chimiques, médicaments, etc., et des chambres de pression pour les appareils respiratoires comprenant une plaque de base (1), une plaque supérieure (2) et un moyen de dilatation ou joint élastique (3) ménagé entre la plaque de base (1) et la plaque supérieure (2), et un support (6) qui est relié rigidement à la partie centrale de la plaque supérieure (2) et qui fait saillie dans un évidement (12) ménagé dans la plaque de base (1), caractérisé en ce que les deux moyens de pression (5a,b), qui se présentent sous la forme de leviers distincts, sont reliés à un support (6) pouvant être pivoté manuellement à l'encontre de l'action des moyens élastiques (9) qui sont montés entre les leviers pour pousser les plaques (1 et 2) l'une vers l'autre et déformer ainsi le joint élastique (3) qui vient en prise avec la paroi du récipient (4).

2. Un dispositif selon la revendication 1 caractérisé en ce que le support (6) présente des logements (7a,b) pour recevoir les axes des leviers (8a,b) traversant les moyens de pression (5a,b).

# FIG.1

# FIG.2